# EUROPEAN PATENT SPECIFICATION

(11) **EP 2 232 264 B1**
(45) Date of publication and mention of the grant of the patent: **02.12.2015**
(21) Application number: 07869484.1
(22) Date of filing: 19.12.2007
(51) Int. Cl.: G01N 33/94, G01N 33/53

(54) **IMMUNOSUPPRESSANT DRUG EXTRACTION REAGENT FOR IMMUNOASSAYS**
IMMUNSUPPRESSIVUM-EXTRAKTIONSREAGENS FÜR IMMUNTESTVERFAHREN
RÉACTIF D'EXTRACTION D'UN IMMUNOSUPPRESSEUR POUR DOSAGES IMMUNOLOGIQUES

(43) Date of publication of application: 29.09.2010
(73) Proprietor: ABBOTT LABORATORIES, Abbott Park, IL 60064-3500 (US)
(72) Inventor: GRENIER, Frank, C., Libertyville Illinois 60048 (US); WORKMAN, Ryan, F., Gurnee, IL 60031 (US); SYED, Hina, N., Gurnee Illinois 60031 (US); ALI, Salman, Hoffman Estates Illinois 60169 (US)
(74) Representative: Modiano, Micaela Nadia
(86) International application number: PCT/US2007/088056
(87) International publication number: WO 2009/078875

(56) References cited:
- EP-A1- 0 471 295
- WO-A1-2008/082982
- US-A- 5 489 668
- US-A- 5 990 150
- US-A1- 2004 102 429
- US-B1- 7 790 928
- US-B2- 7 883 855
- FDA U.S. Food and Drug Administration: "Premarket Notification ARCHITECT Sirolimus", 510(k) Clearances K070822 , October 2007 (2007-10), pages 1-6,1-2,1 of 1, XP002625226, Retrieved from the Internet: URL:http://www.accessdata.fda.gov/cdrh_doc s/pdf7/K070822.pdf [retrieved on 2010-02-25]
- COLANTONIO ET AL: "Comparison of the CEDIA(R) and MEIA(R) assays for the measurement of sirolimus in organ transplant recipients", CLINICAL BIOCHEMISTRY, ELSEVIER INC, US, CA, vol. 40, no. 9-10, 1 June 2007 (2007-06-01), pages 680-687, XP022101720, ELSEVIER INC, US, CA ISSN: 0009-9120, DOI: 10.1016/J.CLINBIOCHEM.2007.02.004
- BOUZAS ET AL: "Determination of everolimus in whole blood using the Abbott IMx(R) sirolimus microparticle enzyme immunoassay", CLINICAL BIOCHEMISTRY, vol. 40, no. 1-2, 30 December 2006 (2006-12-30), pages 132-136, XP005819440, ELSEVIER INC, US, CA ISSN: 0009-9120, DOI: 10.1016/J.CLINBIOCHEM.2006.08.005
- SIMAMORA P ET AL: "Solubilization of rapamycin", INTERNATIONAL JOURNAL OF PHARMACEUTICS, vol. 213, no. 1-2, 1 February 2001 (2001-02-01), pages 25-29, XP002571642, ELSEVIER BV, NL ISSN: 0378-5173, DOI: 10.1016/S0378-5173(00)00617-7
- F. Grenier: "Multi-site Evaluation of the Sirolimus Assay* on the Abbott ARCHITECT Analyzer", Handout for the poster presented at the Americn Association for clincial chamistry Annual Meeting 2006 American Association for Clinical Chemistry Annual Meeting, 1 January 2006 (2006-01-01), XP55037834, Chicago, Illinois Retrieved from the Internet: URL:http://latinoamerica.abbottdiagnostics .com/ciencia/pdf/27.pdf [retrieved on 2012-09-11]
- KOHASHI K ET AL: "Fluorescence reaction of bilirubin with zinc ion in dimethyl sulfoxide and its application to assay of total bilirubin in serum", ANALYTICA CHIMICA ACTA, ELSEVIER, AMSTERDAM, NL, vol. 365, no. 1-3, 5 June 1998 (1998-06-05), pages 177-182, XP002347632, ISSN: 0003-2670, DOI: 10.1016/S0003-2670(97)00666-1
- "Storing cells by freezing", abcam protocol, 1 January 2012 (2012-01-01), XP055193312, [retrieved on 2015-06-03]
- "Common cryoprotectants", ATCC, 1 January 2012 (2012-01-01), XP055193313, [retrieved on 2015-06-03]
- "Freezing and Thawing cells", Salk institute, 1 January 2012 (2012-01-01), XP055193315, [retrieved on 2015-06-03]
- "Rimso product information", , 1 January 2012 (2012-01-01), XP055193316, [retrieved on 2015-06-03]
- "Cryopreservation and storage of cells", , 1 January 2010 (2010-01-01), XP055193317, [retrieved on 2015-06-03]
- "Protocol for freezing cells", , 1 January 2012 (2012-01-01), XP055193318, [retrieved on 2015-06-03]

## Description

### TECHNICAL FIELD

This invention relates to diagnostic immunoassays to determine the concentration levels of an immunosuppressant drug in a patient blood sample, and in particular relates to use of an improved immunosuppressant drug extraction reagent composition.

### BACKGROUND

Many analytes of clinical interest are taken up by cells or become complexed with one or more other components of the test sample. Accordingly, to obtain an accurate measurement of the amount of analyte present in the sample, it is preferable to treat the sample, and/or conduct the assay under conditions, such that the analyte is released from the cells or other component(s) for detection in the assay.

For example, immunosuppressant drugs such as sirolimus (also known as rapamycin), tacrolimus, everolimus, temsorolimus and cyclosporine are effective for the treatment of organ or tissue rejection following transplant surgery, of graft versus host disease and of autoimmune diseases in humans. During immunosuppressant drug therapy, monitoring the blood concentration levels of the immunosuppressant is an important aspect of clinical care because insufficient drug levels lead to graft (organ or tissue) rejection and excessive levels lead to undesired side effects and toxicities. Blood levels of immunosuppressant are therefore measured so drug dosages can be adjusted to maintain the drug level at the appropriate concentration. Diagnostic assays for determination of immunosuppressant blood levels have thus found wide clinical use.

Initially, the immunosuppressant must be extracted and separated from the other components of the patient sample. The bulk of the immunosuppressant drug in the patient sample is present in a complex with various "carrier" molecules, such as binding proteins. Sirolimus, tacrolimus and cyclosporine are found predominately in the red blood cells of patient specimens and are associated with specific binding proteins, FKBP for sirolimus and tacrolimus, and cyclophilin for cyclosporine. To ensure an accurate measurement of the total drug concentration in the specimen, the drug bound to the binding proteins is preferably liberated prior to quantitation. Following its extraction from the binding proteins, the drug can be measured in a number of different ways, including by immunoassay or chromatography with absorbance or mass spectrophotometric detection.

Extraction of sirolimus from its binding proteins in blood is often accomplished by treatment with organic solvents, such as, acetonitrile, methanol, or diethyl ether. These solvents denature the binding proteins and liberate the drug. The use of organic solvents can be problematic, however, when an immunoassay is subsequently used to detect the liberated drug because most organic solvents that will quickly and completely denature the binding proteins are not compatible with immunoassays. They are either too harsh or they create a biphasic sample. Methanol has typically been employed to extract sirolimus, tacrolimus or cyclosporine from blood specimens before immunoassay. However, a careful balance must be achieved such that the methanol concentration is sufficient to liberate the drug from the binding protein, but not so great as to interfere with the subsequent immunoassay. The use of methanol and other typically used organic solvents create an additional problem because these solvents have higher vapor pressure than water. As a result, the extraction supernatant containing the immunosuppressant drug evaporates quickly which causes inaccuracy in the measurement of the drug concentration. The widely used methanol or acetonitrile solvents also create handling and disposal issues for the laboratories.

Immunoassays for immunosuppressant drugs are available in a variety of formats, but all use the binding of an antibody or binding protein (e.g., FKBP) to the immunosuppressant drug. A commonly used immunoassay is an assay which involves the binding of a first antibody to the immunosuppressant and the binding of labeled immunosuppressant (e.g., acridinium-sirolimus) to the remaining free antibody binding sites, followed by quantitation by detection of the label. The effectiveness of these immunoassays is affected by the particular extraction and denaturating solvent for the immunosuppressant that is used.
D1 - WO 2008/082982 relates to immunoassays for immunosuppressant drugs carried out under high salt conditions to achieve improved sensitivity.
D2 - relates to a premarket notification from Fujirebio Diagnostics providing safety and efficacy information to the United States Federal Drug Administration (FDA) regarding Abbott's IMx^{®} ARCHITECT Sirolimus Microparticle Enzyme Immunoassay.
D3 - Grenier, et al. "Multi-site Evaluation of the Sirolimus Assay* on the AbbottARCHITECT® Analyzer," American Association for Clinical Chemistry Annual Meeting, July 23 - 27, 2006, relates to a study that evaluates the performance of the ARCHITECT Sirolimus assay at 3 sites.
D4-Kohashi, et al., Analytica Chimica Acta 365 (1998) 177-182, relates to a fluorimetric method for the determination of total bilirubin in serum.

It is an object of the invention to provide for use with immunoassays an improved immunosuppressant drug extraction reagent composition that has a low vapor pressure, miscibility with water, sufficient immunosupressant denaturing power and compatibility with immunoassay reagents. Such an extraction reagent composition would be advantageous as well for non-immunoassay methods (e.g., chromatographic determinations) because the lower vapor pressure, sufficient denaturing power and water miscibility would make these methods easier to use.

### SUMMARY

Disclosed herein is an improved extractive reagent composition and methods for extracting an immunosuppressant drug, such as sirolimus, tacrolimus, everolimus, temsolorimus, zotarolimus, cyclosporine or analogs thereof, from blood samples while yielding a test sample extract that has low vapor pressure and is compatible with immunoassay components. The reagent composition comprises dimethyl sulfoxide (DMSO), at least one divalent metal salt and water. The preferred reagent composition of the disclousre comprises DMSO and at least one of zinc sulfate, zinc acetate, zinc nitrate, zinc chloride, cadmium sulfate, copper sulfate and mixtures of two or more of these metal salts. A more preferred reagent composition comprises DMSO, the metal salt and at least one glycol having from two to six carbon atoms, which is preferably at least one of ethylene glycol (EG), propylene glycol (PG) or mixtures of EG and PG. Although DMSO, EG and PG are low vapor pressure solvents that are miscible in water and are in routine laboratory use, they have not been employed as protein denaturants but rather have often been added to protein and cell mixtures as stabilizing agents. In contrast, we have found that in the presence of divalent metal cations, DMSO at higher concentrations can serve as an effective protein denaturant to liberate immunosuppressant drugs. In addition, in the presence of divalent cations, lower concentrations of DMSO when mixed with EG or PG can also serve as an effective protein denaturant even though the concentrations of the solvents when used independently are not denaturing. The test sample extracts resulting from use of each of these combinations have low vapor pressure and are compatible with immunochemistry assays.

The disclousre also comprises a method for detecting concentration level of an immunosuppressant drug in a test sample comprising the steps of: (a) combining an extractive reagent composition comprising DMSO, a divalent metal salt and water with the test sample to form a test sample extract; (b) combining the test sample extract with at least one antibody or protein that is immunologically reactive with an immunosuppressant drug to form an assay mixture; (c) incubating the assay mixture under conditions suitable for formation of complexes between the antibody and the immunosuppressant drug, if any, which is present in the sample; and (d) detecting the presence of any complexes formed. Detection of the presence of complexes in step (d) can be carried out using an immunosuppressant to which a signal-generating compound has been attached to bind to the remaining free antibody binding sites on the analyte. A further aspect provides that detection of the presence of complexes in step (d) is carried out using a detectable antibody that binds to the complexes formed in step (c).

The disclosure also comprises a reagent kit for an assay for blood levels of an immunosuppressant drug comprising a container containing the extractive reagent composition comprising DMSO, at least one divalent metal salt and water, and more preferably also comprising ethylene glycol, propylene glycol, or any suitable glycol analog or mixtures thereof. Preferably, the reagent kit further comprises a second container with at least one antibody or protein specific for the immunosuppressant drug. More preferably, the reagent kit contains a third container containing a control composition comprising an immunosuppressant drug, for example, an immunosuppressant selected from the group consisting of sirolimus, tacrolimus, everolimus, zotarolimus, cyclosporine and analogs thereof.

The invention provides:
1) A method for assessment of concentration of an immunosuppressant drug in a human blood sample comprising the steps of: (a) combining an extractive reagent composition comprising at least 50% by volume DMSO a glycol selected from ethylene glycol, propylene glycol, a glycol having from two to six carbon atoms or mixtures thereof, and from 30 mM to 75 mM of a metal salt of zinc with a human blood sample and water to form a test sample extract; (b) combining at least one antibody or protein capable of binding to an immunosuppressant drug with the test sample extract to form a test mixture, wherein the immunosuppressant drug is selected from the group consisting of sirolimus, tacrolimus, everolimus, zotarolimus and cyclosporine; (c) incubating the text mixture under conditions suitable for formation of complexes between the antibody and the immunosuppressant drug, if any, which is present in the sample and is immunologically reactive with the antibody; and (d) detecting the presence of any complexes formed.
2) A method for assessment of concentration of an immunosuppressant drug in a human blood sample comprising contacting a human blood sample with an extractive reagent composition comprising at least 50% by volume dimethyl sulfoxide, a glycol having from two to six carbon atoms or mixtures thereof, and from 30 mM to 75 mM of a metal salt of zinc and water to produce a solid phase and a supernatant phase, separating .the supernatant phase, and analyzing the supernatant phase by immunoassay to determine concentration of a immunosuppressant drug, wherein the immunosuppressant drug is selected from the group consisting of sirolimus, tacrolimus, everolimus, zotarolimus and cyclosporine.
3) The method of item 1 further comprising separating any solid phase resulting from contact of the human blood sample with the extractive reagent composition from any resulting supernatant phase and analyzing the supernatant phase for an immunosuppressant drug.
4) The method of items 1 through 3, wherein the glycol comprises ethylene glycol (EG), propylene glycol (PG), or mixtures of EG and PG.
5) The method of any of items 1 through 4 wherein the metal salt of zinc comprises zinc sulfate, zinc acetate, zinc nitrate, zinc chloride, or mixtures of two or more of these metal salts.
6) The method of any of items 1 through 5 wherein the extractive reagent composition is contacted with the blood sample at a temperature of at least 30 degrees centigrade.
7) The method of any of items 1 through 6 wherein the extractive reagent composition has a vapor pressure of less than water vapor pressure at 20 degrees centigrade and normal atmospheric pressure.
8) An extractive reagent composition for extraction of an immunosuppressant drug from a blood sample, the composition comprising at least 50% by volume dimethyl sulfoxide, a glycol selected from ethylene glycol, propylene glycol, a glycol having from two to six carbon atoms or mixtures thereof, and from 30 mM to 75 mM of a metal salt of zinc and water, wherein the immunosuppressant drug is selected from the group consisting of sirolimus, tacrolimus, everolimus, zotarolimus and cyclosporine.
9) The extractive reagent of item 8, wherein the glycol comprises ethylene glycol (EG), propylene glycol (PG), or mixtures of EG and PG.
10) The extractive reagent composition of item 8 wherein the extractive reagent composition comprises a metal salt of zinc selected from the group consisting of zinc sulfate, zinc acetate, zinc nitrate, zinc chloride, or mixtures of two or more of these metal salts.
11) A test kit comprising separate containers each containing a component selected from (a) at least one antibody or protein capable of binding specifically to at least one immunosuppressant drug selected from the group consisting of sirolimus, tacrolimus, everolimus, zotarolimus and cyclosporine; and (b) an extractive reagent composition comprising at least 50% dimethyl sulfoxide by volume of the extractive reagent composition a glycol selected from ethylene glycol, propylene glycol, a glycol having from two to six carbon atoms or mixtures thereof, and from 30 mM to 75 mM of a metal salt of zinc and water.
12) The test kit of item 11, wherein the glycol comprises ethylene glycol (EG), propylene glycol (PG), or mixtures of EG and PG.
13) The test kit of item 11 or 12 further comprising a container containing a control composition comprising at least one immunosuppressant drug selected from the group consisting of sirolimus, tacrolimus, everolimus, zotarolimus and cyclosporine.
14) The test kit of item 11-13 wherein the zinc salt comprises zinc sulfate, zinc acetate, zinc nitrate or zinc chloride.
15) The test kit of item 11, wherein said test kit comprising separate containers each containing a component selected from (a) at least one antibody or protein capable of binding specifically to at least one immunosuppressant drug selected from the group consisting of sirolimus, tacrolimus, everolimus and cyclosporine; and (b) an extractive reagent composition further comprising 30% - 33% ethylene glycol, propylene glycol or mixtures thereof by volume of the extractive reagent composition, and comprising zinc sulfate; and a control composition comprising at least one immunosuppressant drug selected from the group consisting of sirolimus, tacrolimus, everolimus, zotarolimus and cyclosporine.

The invention has significant capability to provide higher sensitivity immunoassays for determining blood concentration levels of sirolimus, tacrolimus, everolimus, temsorolimus, zotarolimus and cyclosporine. The inventive extractive reagent concentration allows more accurate measurement of the drug levels, while providing better ease of use for the clinical laboratory.

### BRIEF DESCRIPTION OF THE DRAWINGS

Figure 1 shows experimental results on extraction of sirolimus from blood samples using DMSO and zinc sulfate.
Figure 2 shows experimental results on extraction of sirolimus from blood samples using DMSO and varying amounts of zinc sulfate.
Figure 3 shows experimental results on extraction of sirolimus from blood samples using DMSO, EG and zinc sulfate.
Figure 4 shows experimental results on heating the extraction mixture from blood samples and the extractive reagent composition of the invention.
Figure 5 shows a comparison of sample extract evaporation rates resulting from use of a prior art methanol denaturant and from use of the invention.

### DETAILED DESCRIPTION

### I. General

The disclosure comprises extractive reagent compositions useful for the extraction and denaturation of immunosuppressant drugs such as sirolimus from a blood sample; methods for the quantification of immunosuppressant drug levels using the extractive reagent compositions of the disclosure and diagnostic kits comprising the extractive reagent compositions of the disclosure. Preferred methods of the disclosure comprise immunoassays that use immunoreactive specific binding members, such as monoclonal or polyclonal antibodies, or binding proteins (e.g., FKBP) for the formation of complexes with the immunosuppressant drug analyte.

### Definitions

Terms used in the claims and specification are defined as set forth below unless otherwise specified.

An "immunosuppressant drug" or "immunosuppressant", as used herein, refers to a therapeutic compound, either small molecule or antibody based, that has the same or similar chemical structure to either rapamycin (sirolimus), or cyclosporine, also known as cyclosporine A. Any known or hereafter developed analog of either rapamycin or cyclosporine is considered an immunosuppressant herein. Preferred immunosuppressants include sirolimus, tacrolimus, everolimus, temsorolimus, zotarolimus and cyclosporine. Tacrolimus and cyclosporine are calcineurin inhibitors that suppress early activation of the immune system's T lymphocytes through inhibition of cytokines such as interleukin 2. In contrast, the primary target of sirolimus, everolimus and zotarolimus is mammalian target of rapamycin (mTOR), a specific cell-cycle regulatory protein. The inhibition of mTOR leads to suppression of cytokine-driven T-lymphocyte proliferation.

The chemical formula of cyclosporine is in Formula A. The chemical formula of sirolimus (rapamycin) is in Formula B. The chemical formula of the structural difference of everolimus (RAD) from sirolimus is in Formula C.

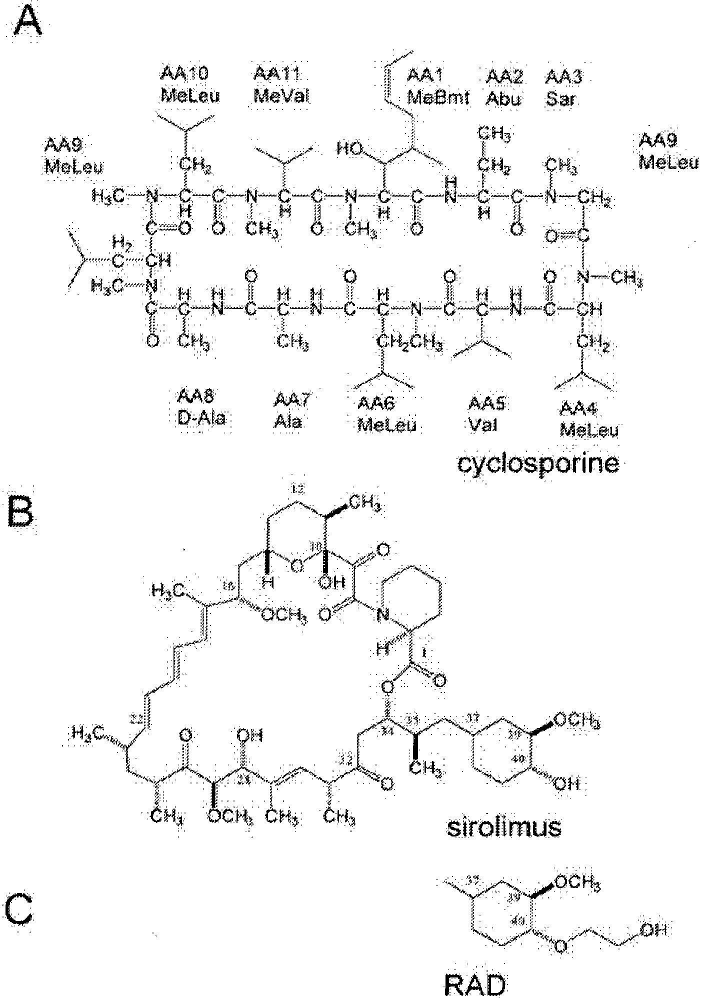

Numerous derivatives or analogs of cyclosporine have been prepared. The disclosure comprises extractive reagents, extractive methods, assays and assay kits for cyclosporine or any of its analogs. Extractive reagents also are described in the literature as lysis reagents.

Numerous derivatives or analogs of rapamycin have been prepared. For example, these include the preparation of ester mono- and di-ester derivatives of rapamycin (PCT International Application WO 92/05179), 27-oximes of rapamycin (European Patent EP 0 467606); 42-oxo analog of rapamycin (U.S. Patent No. 5,023,262); bicyclic rapamycins (U.S. Patent No. 5,120,725); rapamycin dimers (U.S. Patent No. 5,120,727); silyl ethers ofrapamycin (U.S. Patent No. 5,120,842); and arylsulfonates and sulfamates (U.S. Patent No. 5,177,203). Rapamycin was recently synthesized in its naturally occurring enantiomeric form (K. C. Nicolaou et al., J. Am. Chem. Soc., 1993, 115, 4419-4420; S. L. Schreiber., J. Am. Chem. Soc., 1993, 115, 7906-7907; S. J. Danishefsky, J. Am. Chem, Soc., 1993, 115, 9345-9346. The disclosure comprises extractive reagents, extractive methods, assays and assay kits for rapamycin or any of its analogs.

Another immunosuppressant analog of rapamycin is FK-506, also known as tacrolimus, which was isolated from a strain of *S. tsukubaensis*. FK-506's chemical formula is published in European Patent EP 0 293 892 B1. Analogs of FK-506 include the related natural products FR-900520 and FR-900523, which differ from FK-506 in their alkyl substituent at C-21, and were isolated from *S. hygroscopicus yakushimnaensis.* Another analog, FR-900525, produced by *S. tsukubaensis*, differs from FK-506 in the replacement of a pipecolic acid moiety with a proline group. The disclosure comprises extractive reagents, extractive methods, assays and assay kits for FK-506 or any of its analogs. Temsorolimus is another ester derivative of sirolimus which can be monitored with the disclosure

ABT-578 [40-epi-(1-tetrazolyl)-rapamycin], known better today as zotarolimus, is a semi-synthetic macrolide triene antibiotic derived from rapamycin. Zotarolimus' structure is shown in Formula D.

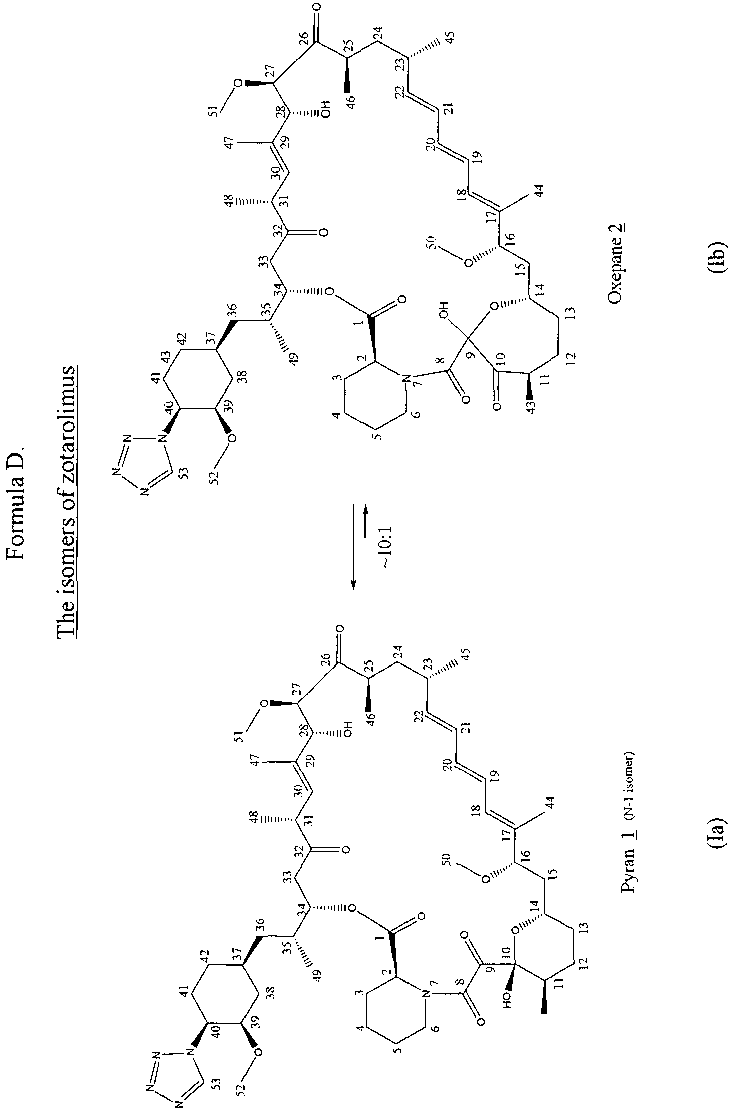

The disclosure comprises extractive reagents, extractive methods, assays and assay kits for zotarolimus or any of its analogs. As used herein with reference to an immunosuppressant drugs, the term "structurally similar" indicates that the drugs have sufficiently similar structures that the drugs bind competitively to at least one common binding partner (e.g., a binding protein).

As used herein, an "antibody" refers to a protein consisting of one or more polypeptides substantially encoded by immunoglobulin genes or fragments of immunoglobulin genes. This term encompasses polyclonal antibodies, monoclonal antibodies, and fragments thereof, as well as molecules engineered from immunoglobulin gene sequences. The recognized immunoglobulin genes include the kappa, lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively.

A typical immunoglobulin (antibody) structural unit is known to comprise a tetramer. Each tetramer is composed of two identical pairs of polypeptide chains, each pair having one "light" (about 25 kD) and one "heavy" chain (about 50 - 70 kD). The N-terminus of each chain defines a variable region of about 100 to 110 or more amino acids primarily responsible for antigen recognition. The terms "variable light chain (VL)" and "variable heavy chain (VH)" refer to these light and heavy chains respectively.

Antibodies exist as intact immunoglobulins or as a number of well-characterized fragments produced by digestion with various peptidases. Thus, for example, pepsin digests an antibody below the disulfide linkages in the hinge region to produce F(ab')2, a dimer of Fab which itself is a light chain joined to VH-CH1 by a disulfide bond. The F(ab')2 may be reduced under mild conditions to break the disulfide linkage in the hinge region thereby converting the (Fab')2 dimer into a Fab' monomer. The Fab' monomer is essentially a Fab with part of the hinge region (see, Fundamental Immunology, W.E. Paul, ed., Raven Press, N.Y. (1993), for a more detailed description of other antibody fragments). While various antibody fragments are defined in terms of the digestion of an intact antibody, one of skill will appreciate that such Fab' fragments may be synthesized de novo either chemically or by utilizing recombinant DNA methodology.

Thus, the term "antibody," as used herein, also includes antibody fragments either produced by the modification of whole antibodies or synthesized de novo using recombinant DNA methodologies. The term "antibody" also encompasses single chain antibodies (antibodies that exist as a single polypeptide chain), more preferably single chain Fv antibodies (sFv or scFv), in which a variable heavy and a variable light chain are joined together (directly or through a peptide linker) to form a continuous polypeptide. The single chain Fv antibody is a covalently linked VH-VL heterodimer which may be expressed from a nucleic acid including VH- and VL-encoding sequences either joined directly or joined by a peptide-encoding linker (Huston, et al. (1988) Proc. Nat. Acad. Sci. USA, 85: 5879-5883). While the VH and VL are connected to each as a single polypeptide chain, the VH and VL domains associate non-covalently. The scFv antibodies and a number of other structures convert the naturally aggregated, but chemically separated, light and heavy polypeptide chains from an antibody V region into a molecule that folds into a three dimensional structure substantially similar to the structure of an antigen-binding site are known to those of skill in the art (see e.g., U.S. Patent Nos. 5,091,513, 5,132,405, and 4,956,778).

"Analyte," as used herein, refers to the substance to be detected, which may be suspected of being present in the test sample. The analyte can be any substance for which there exists a naturally occurring specific binding partner or for which a specific binding partner can be prepared. Thus, an analyte is a substance that can bind to one or more specific binding partners in an assay.

A "binding partner," as used herein, is a member of a binding pair, i.e., a pair of molecules wherein one of the molecules binds to the second molecule. Binding partners that bind specifically are termed "specific binding partners." In addition to the antigen and antibody binding partners commonly used in immunoassays, other specific binding partners can include biotin and avidin, carbohydrates and lectins, complementary nucleotide sequences, effector and receptor molecules, cofactors and enzymes, enzyme inhibitors and enzymes, and the like. Immunoreactive specific binding partners include antigens, antigen fragments, antibodies and antibody fragments, both monoclonal and polyclonal, and complexes thereof, including those formed by recombinant DNA methods.

The term "specific binding" is defined herein as the preferential binding of binding partners to another (e.g., a polypeptide and a ligand (analyte), two polypeptides, a polypeptide and nucleic acid molecule, or two nucleic acid molecules) at specific sites. The term "specifically binds" indicates that the binding preference (e.g., affinity) for the target molecule/sequence is at least 2-fold, more preferably at least 5-fold, and most preferably at least 10- or 20-fold over a non-specific target molecule (e.g. a randomly generated molecule lacking the specifically recognized site(s)).

An antibody that specifically binds an immunosuppressant drug is said to be "specific for" that immunosuppressant drug.

The term "capture agent" is used herein to refer to a binding partner that binds to analyte, preferably specifically. Capture agents can be attached to a solid phase. As used herein, the binding of a solid phase-affixed capture agent to analyte forms a "solid phase-affixed complex."

The term "labeled detection agent" is used herein to refer to a binding partner that binds to analyte, preferably specifically, and is labeled with a detectable label or becomes labeled with a detectable label during use in an assay.

A "detectable label" includes a moiety that is detectable or that can be rendered detectable.

As used with reference to a labeled detection agent, a "direct label" is a detectable label that is attached, by any means, to the detection agent.

As used with reference to a labeled detection agent, an "indirect label" is a detectable label that specifically binds the detection agent. Thus, an indirect label includes a moiety that is the specific binding partner of a moiety of the detection agent. Biotin and avidin are examples of such moieties that are employed, for example, by contacting a biotinylated antibody with labeled avidin to produce an indirectly labeled antibody.

As used herein, the term "indicator reagent" refers to any agent that is contacted with a label to produce a detectable signal. Thus, for example, in conventional enzyme labeling, an antibody labeled with an enzyme can be contacted with a substrate (the indicator reagent) to produce a detectable signal, such as a colored reaction product.

The term "test sample" refers to a component, tissue or fluid of an animal's body that is the source of the immunosuppressant drug analyte. These components, tissues and fluids include human and animal body fluids such as whole blood, serum, plasma, synovial fluid, cerebrospinal fluid, urine, lymph fluids, and various external secretions of the respiratory, intestinal and genitourinary tracts, tears, saliva, milk, white blood cells, myelomas and the like; biological fluids such as cell culture supernatants; fixed tissue specimens; and fixed cell specimens. Preferably, the test sample is a human peripheral blood sample.

### II. Extractive Reagent Compositions

The improved extractive reagent compositions of the disclosure comprise dimethyl sulfoxide (DMSO), at least one divalent metal salt and water. The preferred reagent composition of the disclosure comprises DMSO and at least one of zinc sulfate, zinc acetate, zinc nitrate, zinc chloride, cadmium sulfate and copper sulfate. A more preferred reagent composition comprises DMSO, at least one of ethylene glycol and propylene glycol, or any suitable glycol analog and the metal salt. The preferred compositions of the disclosure have vapor pressures less than water vapor pressure, as measured at 20 degrees centigrade and one atmosphere pressure, and are miscible with water.

Any suitable divalent metal salt that does not precipitate from the reagent composition of the disclosure can be used and salts of zinc are preferred. Exemplary suitable divalent metals include zinc, copper and cadmium. Applicants have not exhaustively tested all possible divalent metal cations, but have determined that tin and manganese sulfates were not suitable at the concentrations tested. The anion of the metal salt can be any suitable anion, including halides, nitrates, sulfates, sulfides, phosphates and acetates. The preferred metal salt is zinc sulfate.

The DMSO concentration, when used without EG or PG, in the extractive reagent composition is at least about 50%, and preferably at least about 80%, up to about 95% by volume of the extractive reagent composition. The metal salt concentration is at least about 5 mM and concentrations up to about 400 mM can be used. A preferred concentration range for the zinc salt is from about 30 to about 75 mM. Use of high salt concentrations, for example above about 75 mM, might require use of a chelating compound, such as ethylene diamine tetraacetic acid, in a subsequent assay step to remove the excess metal. The extractive reagent compositions are made by any suitable mixing method to sufficiently mix the DMSO with water and dissolve the metal salt.

Applicants have found that when EG or PG is included in the extractive reagent composition, then lower concentrations of DMSO are more effective. In these preferred compositions, EG, PG or mixtures thereof are present in a concentration of at least about 18%, and preferably about 25% to about 33% by volume of the extractive reagent composition, and DMSO is present in a concentration of at least about 50% by volume of the extractive reagent composition.

### II. Formation of the Test Sample Extract

The methods of the disclosure are generally carried out on test samples derived from an animal, preferably a mammal, and more preferably a human.

The methods of the disclosure can be carried out using any sample that may contain the analyte of interest (e.g., an immunosuppressant drug), such as a blood sample.

The test sample extract is formed by any mixing technique at any desirable temperature to contact any chosen amount of the blood sample with the extractive reagent composition. Generally about 100 µL to about 600 µL of blood sample is mixed with about 200 µL to about 1200 µL of the extractive reagent composition for up to about five minutes. Preferably, the extraction of the immunosuppressant is accomplished by mixing 150 µL of blood sample with 300 µL of composition and vortexing vigorously for 5 - 10 seconds. Applicants prefer to perform the extraction by heating the extraction mixture to a temperature above room temperature in the range of about 30 degrees centigrade to about 50 degrees centigrade for about five minutes to about sixty minutes. After mixing, the resulting suspension is centrifuged for a suitable time at a suitable revolution rate to produce a supernatant phase and a precipitant phase. Preferably, the mixture is centrifuged at 13,000 rpm for 5 minutes to pellet the precipitant. After the centrifugation, the supernatant is separated using any suitable method. The supernatant is then assayed for the immunosuppressant using any suitable technique, including chromatography and immunoassay.

### III. Immunoassays

In another aspect, the present disclosure relates to immunoassays that can be used for the qualitative identification and/or the quantification of the immunosuppressant drug in a test sample. The disclosure thus comprises a method for detecting concentration level of an immunosuppressant drug in a test sample comprising the steps of: (a) combining an extractive reagent composition comprising DMSO, at least one divalent metal salt with the test sample and water to form a test sample extract; (b) combining at least one antibody or protein capable of binding to an immunosuppressant drug with the test sample extract to form a test mixture; (c) incubating the test mixture under conditions suitable for formation of complexes between the antibody and the immunosuppressant drug, if any, which is present in the sample and is immunologically reactive with the antibody; and (d) detecting the presence of any complexes formed. The immunoassays of the disclosure can be conducted using any format known in the art, such as, but not limited to, a sandwich format, a competitive inhibition format (including both forward or reverse competitive inhibition assays) or in a fluorescence polarization format. The inventors have discovered that an excellent competitive inhibition immunoassay can be performed after using the extractive reagent compositions of the disclosure

In immunoassays for the qualitative or quantitative detection of an immunosuppressant drug in a test sample, at least one antibody or protein that binds to the immunosuppressant drug is contacted with at least one test sample or test sample extract suspected of containing or that is known to contain the immunosuppressant drug to form an antibody-drug or protein-drug immune complex. Any suitable antibodies or binding proteins (e.g., FKBP) that bind to the particular immunosuppressant can be used in the immunoassays disclosed herein.

Antibodies to each of sirolimus, tacrolimus, zotarolimus, cyclosporine and everolimus are known in the art and/or are commercially available, and any of these can be used. It is preferred to use the monoclonal antibody that is a component of Abbott Laboratories' commercially available IMx® Sirolimus assay for measuring sirolimus (Abbott Laboratories, Abbott Park, IL), , or any other Sirolimus assay kit marketed by Abbott Laboratories (e.g., for use on a different commercial automated platform).

An exemplary protocol for producing an antibody specific for an immunosuppressant drug such as sirolimus can be produced as follows. Female RBf/Dnj mice are administered 3 monthly boosts of a sirolimus-27-CMO-tetanus toxoid immunogen followed by an immunization with sirolimus-42-HS-tetanus toxoid preparation on the 4th month. Seven months later, an intrasplenic pre-fusion boost is administered to the animal using the sirolimus-27-CMO-tetanus toxoid immunogen 3 days prior to the fusion. Splenic B-cells are then isolated and used in a standard polyethylene glycol (PEG) fusion with the SP2/0 myeloma. Confluent cultures are screened for anti-sirolimus activity 10-14 days later in a microtiter EIA and positive cultures are then cloned using limiting dilution cloning technique. The resulting clones are isolated and scaled up in IMDM w/FBS (Invitrogen Corp., Carlsbad, CA) tissue culture medium and the secreted antibody is affinity purified using Protein A. The preferred sirolimus antibody described above is also effective for use in immunoassays for sirolimus, everolimus and zotarolimus.

An exemplary preferred antibody for use in immunoassays for tacrolimus is described in M. Kobayashi et al., "A Highly Sensitive Method to Assay FK-506 Levels in Plasma", at pp 23 -29 of "FK-506 A Potential Breakthrough in Immunosuppression", A Transplantation Proceedings Reprint, Supplement 6, Vol. XIX, October, 1987, Editors T. Starzl, L. Makowka and S. Todo, published by Grune & Stratton, Inc., Philadelphia, PA.

An exemplary preferred antibody for use in immunoassays for cyclosporin is the monoclonal antibody that is a component of Abbott Laboratories' commercially available AxSYM® cyclosporine assay for measuring cyclosporine.

The antibody-drug immune complexes can then detected using any suitable technique. For example, the antibody can be labeled with a detectable label to detect the presence of the antibody-drug complex. Any suitable label can be used. The selection of a particular label is not critical, but the chosen label must be capable of producing a detectable signal either by itself or in conjunction with one or more additional substances.

Useful detectable labels, their attachment to antibodies or to other binding proteins and detection techniques therefore are known in the art. Any detectable label known in the art can be used. For example, the detectable label can be a radioactive label, such as, ³H, ¹²⁵I, ³⁵S, ¹⁴C, ³²P, ³³P; an enzymatic label, such as horseradish peroxidase, alkaline peroxidase, glucose 6-phosphate dehydrogenase, etc.; a chemiluminescent label, such as, acridinium derivatives, luminol, isoluminol, thioesters, sulfonamides, phenanthridinium esters, etc.; a fluorescent label, such as, fluorescein (5-fluorescein, 6- carboxyfluorescein, 3'6-carboxyfluorescein, 5(6)-carboxyfluorescein, 6-hexachloro-fluorescein, 6-tetrachlorofluorescein, fluorescein isothiocyanate, etc.), rhodamine, phycobiliproteins, R-phycoerythrin, quantum dots (zinc sulfide-capped cadmium selenide); a thermometric label; or an immuno-polymerase chain reaction label. An introduction to labels, labeling procedures and detection of labels is found in Polak and Van Noorden, *Introduction to Immunocytochemistry,* 2^{nd} ed., Springer Verlag, N.Y.(1997) and in Haugland, *Handbook of Fluorescent Probes and Research Chemi* (1996), which is a combined handbook and catalogue published by Molecular Probes, Inc., Eugene, Oregon. Preferred labels for use with the disclosure are chemiluminescent labels such as acridinium-9-carboxamide. Additional detail can be found in Mattingly, P. G., and Adamczyk, M. (2002) Chemiluminescent N-sulfonylacridinium-9-carboxamides and their application in clinical assays, in Luminescence Biotechnology: Instrument and Applications (Dyke, K. V., Ed.) pp 77-105, CRC Press, Boca Raton.

The detectable label can be bound to the analyte, analyte analog, or antibody either directly or through a coupling agent. An example of a coupling agent that can be used is EDAC (1-ethyl-3-(3-dimethylaminopropyl) carbodiimide, hydrochloride) that is commercially available from Sigma-Aldrich (St. Louis, MO). Other coupling agents that can be used are known in the art. Methods for binding a detectable label to an antibody are known in the art. Additionally, many detectable labels can be purchased or synthesized that already contain end groups that facilitate the coupling of the detectable label to the antibody, such as, N10-(3-sulfopropyl)-N-(3-carboxypropyl)-acridinium-9-carboxamide, otherwise known as CPSP-Acridinium Ester or N10-(3-sulfopropyl)-N-(3-sulfopropyl)-acridinium-9-carboxamide, otherwise known as SPSP-Acridinium Ester.

Alternatively, a second antibody that binds to immunosuppressant and that contains a detectable label can be added to the test sample or test sample extract and used to detect the presence of the antibody-drug complex. Any suitable detectable label can be used in this aspect.

The immunoassays of the disclosure can be conducted using any format known in the art, such as, but not limited to, a sandwich format, a competitive inhibition format (including both forward or reverse competitive inhibition assays) or a fluorescence polarization format. The exemplary formats described below are described in terms of assaying an immunosuppressant drug. However, as those of skill in the art appreciate, the described formats are applicable to any analyte.

In immunoassays for the quantitative detection of an immunosuppressant, such as a preferred sandwich type format, at least two antibodies are employed to separate and quantify the drug in the test sample or test sample extract. More specifically, the at least two antibodies bind to different parts of the drug forming an immune complex which is referred to as a "sandwich". Generally, one or more antibodies can be used to capture the immunosuppressant in the test sample (these antibodies are frequently referred to as a "capture" antibody or "capture" antibodies) and one or more antibodies is used to bind a detectable (namely, quantifiable) label to the sandwich (these antibodies are frequently referred to as the "detection" antibody or "detection" antibodies). In a sandwich assay, it is preferred that both antibodies binding to the drug are not diminished by the binding of any other antibody in the assay to its respective binding site. In other words, antibodies should be selected so that the one or more first antibodies brought into contact with a test sample or test sample extract suspected of containing an immunosuppressant do not bind to all or part of the binding site recognized by the second or subsequent antibodies, thereby interfering with the ability of the one or more second detection antibodies to bind to the drug. In a sandwich assay, the antibodies, preferably, the at least one capture antibody, are used in molar excess amounts of the maximum amount of drug expected in the test sample or test sample extract. For example, from about 5 µg/mL to about 1 mg/mL of antibody per mL of solid phase containing solution can be used.

In one aspect the at least one first capture antibody can be bound to a solid support which facilitates the separation of the first antibody-drug complex from the test sample. The solid support or "solid phase" used in the immunoassay disclosed herein is not critical and can be selected by one skilled in the art. A solid phase or solid support, as used herein, refers to any material that is insoluble, or can be made insoluble by a subsequent reaction. Useful solid phases or solid supports are known to those in the art and include the walls of wells of a reaction tray, test tubes, polystyrene beads, magnetic beads, nitrocellulose strips, membranes, microparticles such as latex particles, sheep (or other animal) red blood cells, and Duracytes® (a registered trademark of Abbott Laboratories, Abbott Park, Ill.), which are red blood cells "fixed" by pyruvic aldehyde and formaldehyde, and others. Suitable methods for immobilizing peptides on solid phases include ionic, hydrophobic, covalent interactions and the like. The solid phase can be chosen for its intrinsic ability to attract and immobilize the capture agent. Alternatively, the solid phase can comprise an additional receptor which has the ability to attract and immobilize the capture agent. The additional receptor can include a charged substance that is oppositely charged with respect to the capture agent itself or to a charged substance conjugated to the capture agent. As yet another alternative, the receptor molecule can be any specific binding member partner which is immobilized upon (attached to) the solid phase and which has the ability to immobilize the capture agent through a specific binding reaction. The receptor molecule enables the indirect binding of the capture agent to a solid phase material before the performance of the assay or during the performance of the assay.

Any solid support known in the art can be used, including but not limited to, solid supports made out of polymeric materials in the forms of wells, tubes or beads. The antibody (or antibodies) can be bound to the solid support by adsorption, by covalent bonding using a chemical coupling agent or by other means known in the art, provided that such binding does not interfere with the ability of the antibody to bind the drug. Moreover, if necessary, the solid support can be derivatized to allow reactivity with various functional groups on the antibody. Such derivatization requires the use of certain coupling agents such as, but not limited to, maleic anhydride, N-hydroxysuccinimide and 1-ethyl-3-(3-dimethylaminopropyl)carbodiimide.

The solid phase may comprise any suitable porous material with sufficient porosity to allow access by detection antibodies and a suitable surface affinity to bind antigens. Microporous structure generally are preferred, but materials with gel structure in the hydrated state may be used as well. Such useful solid supports include but are not limited to nitrocellulose and nylon. It is contemplated that such porous solid supports described herein preferably are in the form of sheets of thickness from about 0.01 to 0.5 mm, preferably about 0.1 mm. The pore size may vary within wide limits, and preferably is from about 0.025 to 15 microns, especially from about 0.15 to 15 microns. The surface of such supports may be activated by chemical processes which cause covalent linkage of the antigen or antibody to the support. The irreversible binding of the antigen or antibody is obtained, however, in general, by adsorption on the porous material by hydrophobic forces.

After the test sample extract suspected of containing or containing the immunosuppressant is brought into contact with the at least one first capture antibody, the resulting mixture is incubated to allow for the formation of a first capture antibody (or multiple antibody)-drug complex. The incubation can be carried out at any suitable pH, including a pH of from about 4.5 to about 10.0, at any suitable temperature, including from about 2°C to about 45°C, and for a suitable time period from at least about one (1) minute to about eighteen (18) hours, preferably from about 4-20 minutes, most preferably from about 17-19 minutes.

After the addition of a detection agent and the formation of a labeled complex, the amount of label in the complex is quantified using techniques known in the art. For example, if an enzymatic label is used, the labeled complex is reacted with a substrate for the label that gives a quantifiable reaction such as the development of color. If the label is a radioactive label, the label is quantified using a scintillation counter. If the label is a fluorescent label, the label is quantified by stimulating the label with a light of one color (which is known as the "excitation wavelength") and detecting another color (which is known as the "emission wavelength") that is emitted by the label in response to the stimulation. If the label is a chemiluminescent label, the label is quantified detecting the light emitted either visually or by using luminometers, x-ray film, high speed photographic film, a CCD camera, etc. Once the amount of the label in the complex has been quantified, the concentration of drug in the test sample can be determined by use of a standard curve that has been generated, e.g., using serial dilutions of immunosuppressant drug of known concentration. Other than using serial dilutions of the drug, the standard curve can be generated gravimetrically, by mass spectroscopy and by other techniques known in the art.

In a preferred forward competitive format, an aliquot of labeled drug or analogue thereof, of a known concentration is used to compete with the drug present in a test sample for binding to the antibody. In a forward competition assay, an immobilized antibody can either be sequentially or simultaneously contacted with the test sample and a labeled drug or drug analogue thereof. The drug or drug analogue can be labeled with any suitable detectable label, including those detectable labels discussed above. In this assay, the capture antibody of the present disclosure can be immobilized on to a solid support using the techniques discussed previously herein. Alternatively, the capture antibody can be coupled to an antibody, such as an antispecies antibody, that has been immobilized on to a solid support, such as a microparticle.

The labeled drug or drug analogue, the test sample extract and the antibody are incubated under conditions similar to those described above in connection with the sandwich assay format. Two different types of antibody-drug complexes are then generated. Specifically, one of the antibody-drug complexes generated contains a detectable label while the other antibody-drug complex does not contain a detectable label. The antibody-drug complex can be, but does not have to be, separated from the remainder of the test sample extract prior to quantification of the detectable label. Regardless of whether the antibody-drug complex is separated from the remainder of the test sample, the amount of detectable label in the antibody-drug complex is then quantified. The concentration of drug in the test sample can then be determined by comparing the quantity of detectable label in the antibody-drug complex to a standard curve. The standard curve can be generated using serial dilutions of the drug of known concentration, by mass spectroscopy, gravimetrically and by other techniques known in the art.

The antibody-drug complex can be separated from the test sample by binding the antibody to a solid support, such as the solid supports discussed above in connection with the sandwich assay format, and then removing the remainder of the test sample from contact with the solid support.

In a reverse competition assay, an immobilized immunosuppressant drug or analogue thereof can either be sequentially or simultaneously contacted with a test sample or test sample extract and at least one labeled antibody or labeled protein. The antibody or protein can be labeled with any suitable detectable label, including those detectable labels discussed above. The drug or drug analogue can be bound to a solid support, such as the solid supports discussed above in connection with the sandwich assay format.

The immobilized drug or drug analogue, test sample or test sample extract, and at least one labeled antibody or labeled protein are incubated under conditions similar to those described above in connection with the sandwich assay format. Two different types of antibody-drug or protein-drug complexes are then generated. Specifically, one of the antibody-drug (or protein-drug) complexes generated is immobilized and contains a detectable label while the other antibody-drug (or protein-drug) complex is not immobilized and contains a detectable label. The non-immobilized antibody-drug complex and the remainder of the test sample or test sample extract are removed from the presence of the immobilized antibody-drug complex through techniques known in the art, such as washing. Once the non-immobilized antibody-drug complex is removed, the amount of detectable label in the immobilized antibody-drug complex is then quantified. The concentration of drug in the test sample can then be determined by comparing the quantity of detectable label in the antibody-drug complex to a standard curve. The standard curve can be generated using serial dilutions of the drug of known concentration, by mass spectroscopy, gravimetrically and by other techniques known in the art.

In a fluorescence polarization assay, in one aspect an antibody or functionally active fragment thereof is first contacted with an unlabeled test sample containing the immunosuppressant drug to form an unlabeled antibody-drug complex. The unlabeled antibody-drug complex is then contacted with a fluorescently labeled drug or analogue thereof. The labeled drug or drug analogue competes with any unlabeled drug in the test sample for binding to the antibody or functionally active fragment thereof. The amount of labeled antibody-drug complex formed is determined and the amount of drug in the test sample determined via use of a standard curve.

In a further aspect of the present disclosure there is disclosed a method for detecting concentration level of an immunosuppressant drug in a test sample comprising the steps of: (a) combining an extractive reagent composition comprising DMSO, at least one divalent metal salt and water with the test sample to form a test sample extract; (b) combining at least one antibody or protein capable of binding to an immunosuppressant drug with the test sample extract to form a mixture; (c) incubating the mixture under conditions suitable for formation of complexes between the antibody and the immunosuppressant drug, if any, which is present in the sample and is immunologically reactive with the antibody; and (d) detecting the presence of any antibody-immunosuppressant drug complexes formed.

### IV. Other Immunosuppressant Assays

Any other alternative measurement method for the concentration of immunosuppressant can also be used with the extractive reagent disclosed herein. For example, the drug content can be determined by a mass spectrometry based method, such as the rapid liquid chromatography-tandem mass spectrometry technique described in N. Brown et al., "Low Hematocrit and Serum Albumin Concentrations Underlie the Overestimation of Tacrolimus Concentrations by Microparticle Enzyme Immunoassay versus Liquid Chromatography-Tandem Mass Spectrometry", Clinical Chemistry. 2005;51:586-592.

### V. Instrumentation

Any suitable instrumentation or automation can be used in the performance of the contact of the extractive reagent composition with the blood sample and in the performance of the drug concentration level assay. It is preferred to carry out the assay in an automated fashion, such as on the ARCHITECT® (a registered trademark of Abbott Laboratories, Abbott Park, Ill.) system, which uses chemiluminescense detection of sandwich hybridization and competitive immunoassays. The assay can also be carried out in a miniaturized format, such as in a Lab-on-a-Chip device and system.

### VI. Immunoassay Kits

In another aspect, the disclosure comprises immunoassay kits for the detection of an immunosuppressant drug of the same or similar chemical structure to either sirolimus or cyclosporine, preferably selected from the group consisting of sirolimus, tacrolimus, everolimus, zotarolimus, cyclosporine and analogs thereof, which kits comprise an extractive reagent composition of the disclosure. These kits may also include an antibody capture agent or antibody indicator reagent useful to carry out a sandwich immunoassay. Preferred kits of the disclosure comprise containers containing, respectively, at least one antibody or protein capable of binding specifically to at least one immunosuppressant drug selected from the group consisting of sirolimus, tacrolimus, everolimus, zotarolimus and cyclosporine; an extractive reagent composition comprising 50 % DMSO by volume of the extractive reagent composition, 30% - 33% EG, PG or mixtures thereof by volume of the extractive reagent composition, water and zinc sulfate at a concentration of at least 5 mM; and a control composition comprising at least one immunosuppressant drug selected from the group consisting of sirolimus, tacrolimus, everolimus, zotarolimus, cyclosporine or analogs thereof.

Any suitable control composition for the particular immunosuppressant drug assay can be included in the kits of the disclosure. The control compositions generally comprise the actual immunosuppressant to be assayed for along with any desirable additives. For example, the control composition for tacrolimus can be the control compositions described in U.S. Patent 5,338,684, Grenier et al.

Kits according to the disclosure can include a solid phase and a capture agent that is affixed to the solid phase or that becomes solid phase-affixed during the assay. In exemplary aspects the solid phase includes one or more microparticles or electrodes. Where such kits are to be employed for conducting sandwich immunoassays, the kits can additionally include a labeled detection agent. In certain aspects the test kit includes at least one direct label, such as acridinium-9-carboxamide. Test kits according to the disclosure can also include at least one indirect label. If the label employed generally requires an indicator reagent to produce a detectable signal, the test kit preferably includes one or more suitable indicator reagents.

Test kits according to the disclosure preferably include instructions for carrying out one or more of the immunoassays of the disclosure. Instructions included in kits of the disclosure can be affixed to packaging material or can be included as a package insert. While the instructions are typically written or printed materials they are not limited to such. Any medium capable of storing such instructions and communicating them to an end user is contemplated by this disclosure. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

Of course, it goes without saying that any of the exemplary formats herein, and any assay or kit according to the disclosure can be adapted or optimized for use in automated and semi-automated systems (including those in which there is a solid phase comprising a microparticle), as described, e.g., in U.S. Patent Nos. 5,089,424 and 5,006,309, and as, e.g., commercially marketed by Abbott Laboratories (Abbott Park, IL) including but not limited to Abbott Laboratories' ARCHITECT®, AxSYM®, IMX®, ABBOTT PRISM®, and Quantum II platforms, as well as other platforms.

Additionally, the assays and kits of the present disclosure optionally can be adapted or optimized for point of care assay systems, including Abbott Laboratories' Point of Care (i-STAT®) electrochemical immunoassay system. Immunosensors and methods of manufacturing and operating them in single-use test devices are described, for example in U.S. Patent 5,063,081 and published U.S. Patent Applications 20030170881, 20040018577, 20050054078, and 20060160164. The following examples are offered to illustrate, but not to limit, the claimed invention.

### EXAMPLE 1

This example illustrates the use of DMSO in combination with zinc sulfate to extract sirolimus from binding proteins in blood samples. The extracted sirolimus is measured with an immunoassay.

The extractive reagent composition was prepared at final concentrations of 86% DMSO, 14% water and 40 mM zinc sulfate. Extraction of the blood sirolimus samples was accomplished by mixing 100 µL of blood sample with 200 µL of the reagent composition and vortexing vigorously for 5 - 10 seconds. The resulting suspension was centrifuged at 13,000 rpm for 5 minutes to pellet the precipitant and the supernatant extract was assayed for sirolimus as follows. The assay was executed on an automated ARCHITECT® i2000® analyzer (Abbott Laboratories, Abbott Park, Illinois) by:
1. Mixing 10 - 40 µL of the sample extract with 50 µL of microparticles coated with goat anti-mouse antibody (from Sigma-Aldrich, St. Louis, Missouri) and mouse anti-sirolimus antibody (prepared as described below).
2. Incubating the reaction mixture for approximately 18 minutes at 33 - 38 degrees C. The sirolimus in the sample binds the anti-sirolimus antibody on the microparticles.
3. Adding 20 µL of acridinium-sirolimus conjugate to the reaction mixture.
4. Incubating the reaction mixture for approximately 4 minutes at 33 - 38 degrees C. The acridinium-sirolimus conjugate binds free anti-sirolimus binding sites.
5. Washing the microparticles with a phosphate buffer.
6. Adding Pre-trigger (acid solution) and Trigger (basic solution) to cause the captured acridinium-sirolimus label to emit light, which is measured by the instrument.

The sirolimus binding antibody was produced as follows: Female RBf/Dnj mice were administered 3 monthly boosts of a sirolimus-27-CMO-tetanus toxoid immunogen followed by an immunization with sirolimus-42-HS-tetanus toxoid preparation on the 4th month. Seven months later, an intrasplenic pre-fusion boost was administered to the animals using the sirolimus-27-CMO-tetanus toxoid immunogen 3 days prior to the fusion. Splenic B-cells were isolated and used in a standard PEG fusion with the SP2/0 myeloma. Confluent cultures were screened for anti-sirolimus activity 10-14 days later in a microtiter EIA, and positive cultures were cloned using limiting dilution cloning technique. Isolated clones were scaled up in IMDM w/FBS (Invitrogen Corp., Carlsbad, CA) tissue culture medium, and secreted antibody was affinity purified using Protein A.

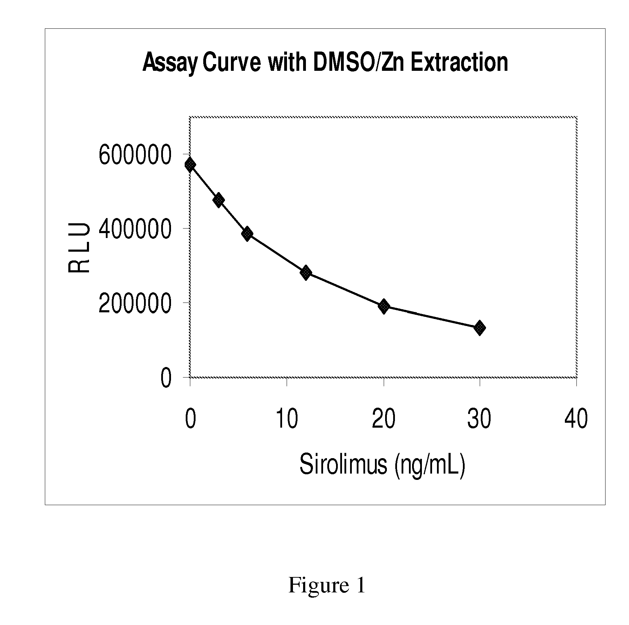

The response curve data are shown in Table 1 and Figure 1 and demonstrate that sirolimus was liberated from binding proteins with the DMSO/zinc sulfate composition.

**Table 1**

| Sirolimus (ng/mL) | RLUs |
|---|---|
| 0 | 573660 |
| 3 | 474794 |
| 6 | 387857 |
| 12 | 279516 |
| 20 | 191208 |
| 30 | 131426 |

Signal measurements in Table 1 are in RLUs (Relative Light Units). RLUs are the designation for the optical unit of measurement utilized on the ARCHITECT® systems. The ARCHITCT optics system is essentially a photomultiplier tube (PMT) that performs photon counting on the light emitted by the chemiluminescent reaction. The amount of light generated by the chemiluminescent reaction is proportional to the amount of acridinium tracer present in the reaction mixture, and thereby allows quantitation of the patient sample analyte that is also proportional to the amount of acridinium remaining in the reaction mixture at the time the chemiluminescent reaction occurs.

The term Relative Light Units comes from the relation of the photon counting to a certain amount of acridinium. Each optics module is calibrated with a set of acridinium standards. When the chemiluminescent reaction occurs, light is emitted and the photons are measured over a 3 second time period. The PMT converts the photons counted to digital signal, which is then sent to a circuit board for processing. The optics circuit board converts the digital signal from the PMT to an analog signal that is proportional to the photons counted, which is in turn proportional to the amount of acridinium present. This analog signal is then further processed to produce an RLU value. This relationship was established to produce a standard for calibration of the optics module, where the different acridinium standards have RLU values assigned to them. So, while the RLU unit itself is arbitrary, it is proportional (i.e., relative) to a certain amount of acridinium.

### EXAMPLE 2

This example illustrates the utility of varying zinc concentrations in the composition.

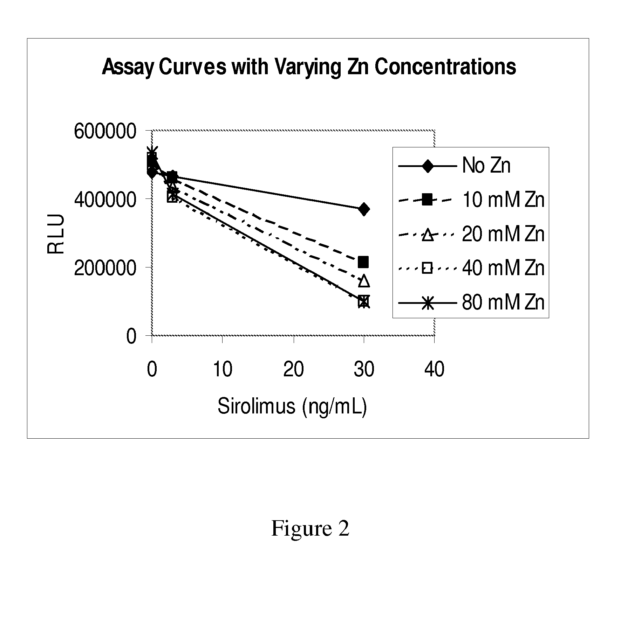

The composition was prepared at varying final concentrations of zinc sulfate (0, 10, 20, 40 and 80 mM) in 90% DMSO and 10% water. Extraction of the blood sirolimus samples was accomplished by mixing 400 µL of blood sample with 800 µL of composition and vortexing vigorously for 5 - 10 seconds. The resulting suspension was centrifuged at 13,000 rpm for 5 minutes to pellet the precipitant and the supernatant was assayed for sirolimus. The sirolimus assay was performed as in Example 1. The test data are shown in Table 2 and Figure 2 and illustrate that the liberation of sirolimus from binding proteins is improved by the addition of the zinc salt. Applicants have also tested and determined that zinc chloride, zinc nitrate, and zinc acetate are also efficacious.

**Table 2**

| | RLU | | | | |
|---|---|---|---|---|---|
| Sirolimus (ng/mL) | No Zn | 10 mM Zn | 20 mM Zn | 40 mM Zn | 80 mM Zn |
| 0 | 477005 | 509875 | 503822 | 517429 | 532760 |
| 3 | 464256 | 461998 | 435120 | 403487 | 414074 |
| 30 | 367821 | 214924 | 161065 | 99300 | 100777 |

### EXAMPLE 3

This example illustrates the utility of different divalent cations in the composition.

The extraction composition was prepared at different final concentrations of metal salts (zinc sulfate, cadmium sulfate, copper sulfate, tin sulfate and manganese sulfate) and DMSO as shown in the table below (remaining volume was water). Extraction of the blood sirolimus samples was accomplished by mixing 200 µL of blood sample with 400 µL of composition and vortexing vigorously for 5 - 10 seconds. The resulting suspension was centrifuged at 13,000 rpm for 5 minutes to pellet the precipitant and the supernatant was assayed for sirolimus. The sirolimus assay was performed as in Example l.The example data are shown in Table 3 and demonstrate that metal salts other than zinc can also be utilized.

**Table 3**

| DMSO | 90% | 90% | 50% | 90% | 90% |
|---|---|---|---|---|---|
| Metal Salt | 40 mM Zn | 50 mM Cd | 50 mM Cu | 20 mM Sn | 20 mM Mn |
| Sirolimus (ng/mL) | RLUs | RLUs | RLUs | RLUs | RLUsl |
| 0 | 550492 | 536394 | 523472 | 517375 | 524178 |
| 3 | 441164 | 413887 | 430245 | 475927 | 469885 |
| 30 | 107957 | 89515 | 126515 | 222690 | 220562 |

### EXAMPLE 4

This example illustrates the utility of varying ratios of DMSO and EG in the extractive reagent composition.

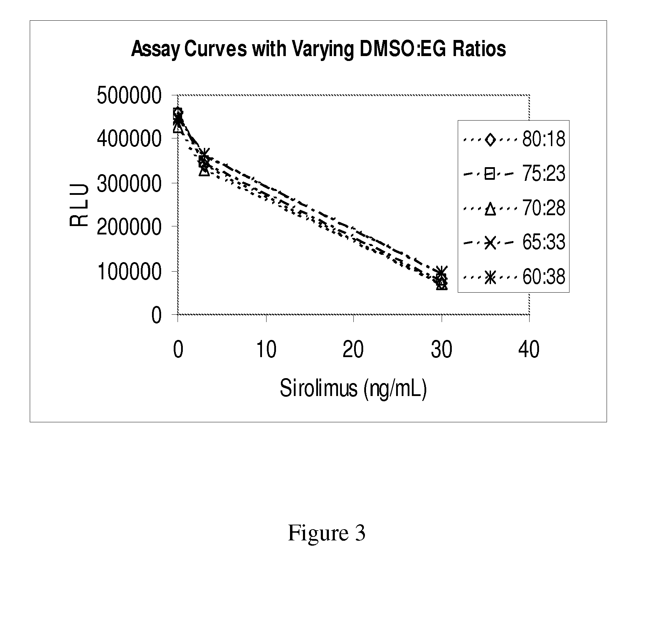

The composition was prepared at different final concentration ratios of DMSO and ethylene glycol (percent DMSO and percent ethylene glycol were 80:18, 75:23, 70:28, 65:33 and 60:33, respectively). All compositions had 2% water and 40 mM zinc sulfate. Extraction of the blood sirolimus samples was accomplished by mixing 150 µL of blood sample with 300 µL of composition and vortexing vigorously for 5 - 10 seconds. The resulting suspension was centrifuged at 13,000 rpm for 5 minutes to pellet the precipitant and the supernatant was assayed for sirolimus. The sirolimus assay was performed as in Example 1. The example data are shown in Table 4 and Figure 3 and demonstrate that sufficient denaturing power can be maintained when the concentration of DMSO is decreased if the ethylene glycol concentration is increased.

**Table 4**

| | RLUs with varying DMSO:EG Ratios | | | | |
|---|---|---|---|---|---|
| Sirolimus (ng/mL) | 80:18 | 75:23 | 70:28 | 65:33 | 60:38 |
| 0 | 457694 | 454223 | 428721 | 450344 | 446482 |
| 3 | 340579 | 346026 | 330247 | 364041 | 363857 |
| 30 | 70582 | 78831 | 72669 | 95479 | 94442 |

Concentrations of DMSO or ethylene glycol that are not at all or only marginally efficacious when used alone (e.g., 65% DMSO or 33% ethylene glycol) are highly effective in combination. Propylene glycol can be substituted for ethylene glycol in the DMSO:ethylene glycol mixtures (data not shown). The DMSO:ethylene glycol mixtures have the additional advantage of increased solubility for zinc sulfate.

### EXAMPLE 5

This example illustrates the improvement in the sirolimus extraction efficiency achieved by heating the extraction mixture.

The DMSO-based composition was prepared at final concentrations of 70% DMSO, 28% ethylene glycol, 2% water and 46 mM zinc sulfate. Extraction of the blood sirolimus samples was accomplished by mixing 150 µL of blood sample with 300 µL of composition, vortexing vigorously for 5 - 10 seconds and incubating at different temperatures for 15 minutes. The resulting suspension was centrifuged at 13,000 rpm for 5 minutes to pellet the precipitant and the supernatant was assayed for sirolimus. The sirolimus assay was performed as in Example 1.

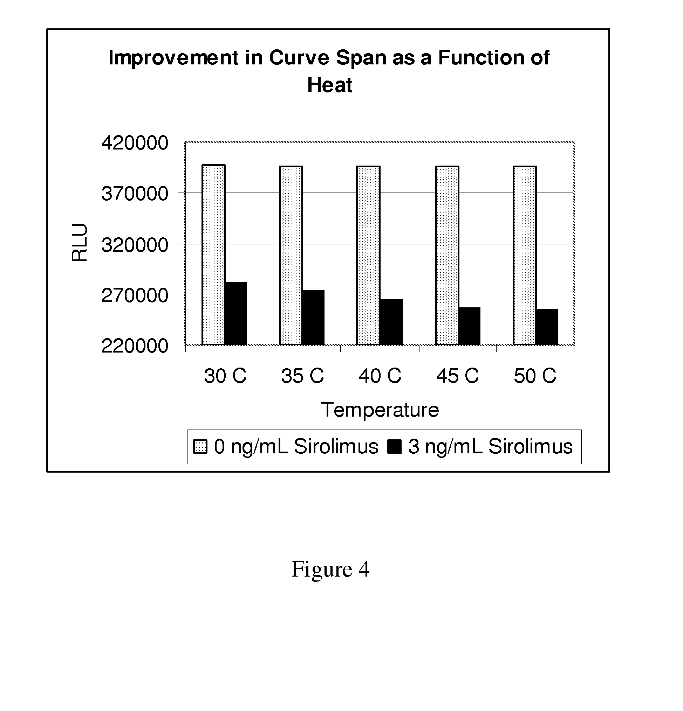

The example data are shown in Table 5 and Figure 4 and demonstrate that more efficient liberation of sirolimus occurs when the extraction mixture is heated (i.e. the difference between the 0 ng/mL calibrator and the 3 ng/mL calibrator increases with increasing temperature).

**Table 5**

| | RLUs as a Function of Temperature | | | | |
|---|---|---|---|---|---|
| | 30 C | 35 C | 40 C | 45 C | 50 C |
| 0 ng/mL Sirolimus | 397353 | 395993 | 396009 | 395694 | 395957 |
| 3 ng/mL Sirolimus | 281375 | 273856 | 264768 | 256660 | 254426 |

### EXAMPLE 6

This example illustrates the minimal evaporation of a DMSO-based extraction composition compared to a typical methanol-based extraction composition.

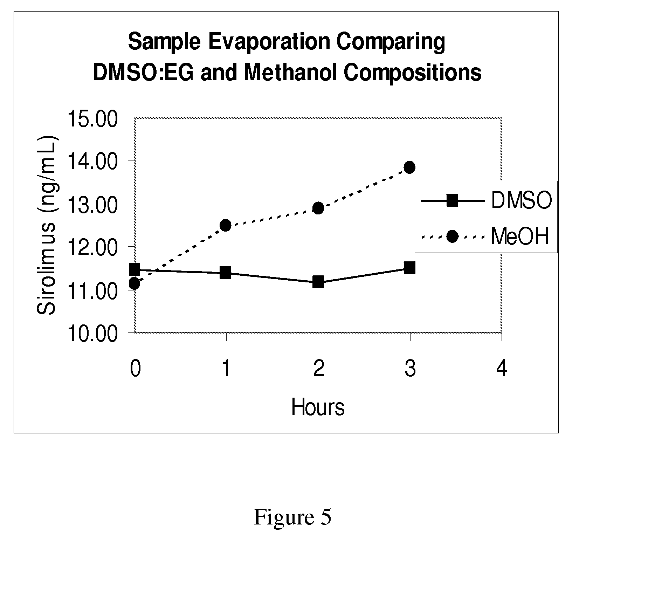

The DMSO-based composition was prepared at final concentrations of 70% DMSO, 28% ethylene glycol, 2% water and 46 mM zinc sulfate. The methanol-based composition was prepared at final concentrations of 80% methanol, 18% ethylene glycol, 2% water and 50 mM zinc sulfate. Extraction of the blood sirolimus samples with either composition was accomplished by mixing 125 µL of blood sample with 250 µL of composition and vortexing vigorously for 5 - 10 seconds. The DMSO-based composition extraction mixture was incubated at 42° C for 10 minutes and the methanol-based composition extraction mixture was incubated at room temperature for 10 minutes. The extraction mixtures were centrifuged at 13,000 rpm for 5 minutes to pellet the precipitant and the supernatants were decanted in the sample cups used in the ARCHITECT® i2000® analyzer. A set of samples was tested for evaporation immediately (time zero) and at 1, 2 and 3 hours after decanting into the sample cup (samples allowed to sit at room temperature, approximately 22° C). The test data are shown in Table 6 and Figure 5 and illustrate the rapid evaporation of methanol-based compositions, which is manifested as higher sirolimus concentrations. In less than 2 hours, the error in the sirolimus concentration had exceeded 10% with the methanol-based composition, but essentially no change in concentration had occurred with the DMSO-composition. The error in the sirolimus concentration would be greater if the humidity was lower or if the incubation temperature was higher (some immunochemistry assay analyzers incubate samples at temperatures near 37° C).

**Table 6**

| | Sirolimus (ng/mL) | |
|---|---|---|
| Hours | DMSO | MeOH |
| 0 | 11.47 | 11.14 |
| 1 | 11.37 | 12.47 |
| 2 | 11.18 | 12.88 |
| 3 | 11.50 | 13.83 |

## Claims

1. A method for assessment of concentration of an immunosuppressant drug in a human blood sample comprising the steps of:
(a) combining an extractive reagent composition comprising at least 50% by volume DMSO, a glycol having from two to six carbon atoms, and from 30 mM to 75 mM of a metal salt of zinc with a human blood sample and water to form a test sample extract;
(b) combining at least one antibody or protein capable of binding to an immunosuppressant drug with the test sample extract to form a test mixture, wherein the immunosuppressant drug is selected from the group consisting of sirolimus, tacrolimus, everolimus, zotarolimus and cyclosporine;
(c) incubating the text mixture under conditions suitable for formation of complexes between the antibody and the immunosuppressant drug, if any, which is present in the sample and is immunologically reactive with the antibody; and
(d) detecting the presence of any complexes formed.

2. A method for assessment of concentration of an immunosuppressant drug in a human blood sample comprising contacting a human blood sample with an extractive reagent composition comprising at least 50% by volume dimethyl sulfoxide, a glycol having from two to six carbon atoms, and from 30 mM to 75 mM of a metal salt of zinc and water to produce a solid phase and a supernatant phase, separating .the supernatant phase, and analyzing the supernatant phase by immunoassay to determine concentration of a immunosuppressant drug, wherein the immunosuppressant drug is selected from the group consisting of sirolimus, tacrolimus, everolimus, zotarolimus and cyclosporine.

3. The method of claim 1 further comprising separating any solid phase resulting from contact of the human blood sample with the extractive reagent composition from any resulting supernatant phase and analyzing the supernatant phase for an immunosuppressant drug.

4. The method of any of claims 1 through 3 wherein the glycol comprises ethylene glycol (EG), propylene glycol (PG) or mixtures of EG and PG.

5. The method of any of claims 1 through 4 wherein the metal salt of zinc comprises zinc sulfate, zinc acetate, zinc nitrate, zinc chloride, or mixtures of two or more of these metal salts.

6. The method of any of claims 1 through 5 wherein the extractive reagent composition is contacted with the blood sample at a temperature of at least 30 degrees centigrade.

7. The method of any of claims 1 through 6 wherein the extractive reagent composition has a vapor pressure of less than water vapor pressure at 20 degrees centigrade and normal atmospheric pressure.

8. An extractive reagent composition for extraction of an immunosuppressant drug from a blood sample, the composition comprising at least 50% by volume dimethyl sulfoxide, a glycol having from two to six carbon atoms, and from 30 mM to 75 mM of a metal salt of zinc and water, wherein the immunosuppressant drug is selected from the group consisting of sirolimus, tacrolimus, everolimus, zotarolimus and cyclosporine.

9. The extractive reagent composition of claim 8 wherein the glycol comprises ethylene glycol (EG), propylene glycol (PG) or mixtures of EG and PG.

10. The extractive reagent composition of claims 8 or 9 wherein the extractive reagent composition comprises a metal salt of zinc selected from the group consisting of zinc sulfate, zinc acetate, zinc nitrate, zinc chloride, or mixtures of two or more of these metal salts.

11. A test kit comprising separate containers each containing a component selected from
(a) at least one antibody or protein capable of binding specifically to at least one immunosuppressant drug selected from the group consisting of sirolimus, tacrolimus, everolimus, zotarolimus and cyclosporine; and
(b) an extractive reagent composition comprising at least 50% dimethyl sulfoxide by volume of the extractive reagent composition, a glycol having from two to six carbon atoms, and from 30 mM to 75 mM of a metal salt of zinc and water.

12. The test kit of claim 11 wherein the glycol comprises ethylene glycol (EG), propylene glycol (PG) or mixtures of EG and PG.

13. The test kit of claims 11 or 12 further comprising a container containing a control composition comprising at least one immunosuppressant drug selected from the group consisting of sirolimus, tacrolimus, everolimus, zotarolimus and cyclosporine.

14. The test kit of claims 11 - 13 wherein the zinc salt comprises zinc sulfate, zinc acetate, zinc nitrate or zinc chloride.

15. The test kit of claim 11, wherein said test kit comprising separate containers each containing a component selected from (a) at least one antibody or protein capable of binding specifically to at least one immunosuppressant drug selected from the group consisting of sirolimus, tacrolimus, everolimus and cyclosporine; and (b) an extractive reagent composition further comprising 30%- 33% ethylene glycol, propylene glycol or mixtures thereof by volume of the extractive reagent composition, and comprising zinc sulfate; and a control composition comprising at least one immunosuppressant drug selected from the group consisting of sirolimus, tacrolimus, everolimus, zotarolimus and cyclosporine.

## Patentansprüche

1. Ein Verfahren zur Einschätzung der Konzentration eines Immunsuppressivums in einer menschlichen Blutprobe, das die folgenden Schritte umfasst:
(a) Kombinieren einer extraktiven Reagenz-Zusammensetzung umfassend mindestens 50 Volumenprozent DMSO, ein Glykol mit von zwei bis sechs Kohlenstoffatomen, und von 30 mM bis 75 mM eines Metallsalzes von Zink mit einer menschlichen Blutprobe und Wasser, um einen Testprobenextrakt zu bilden;
(b) Kombinieren mindestens eines Antikörpers oder Proteins, das in der Lage ist an ein Immunsuppressivum zu binden, mit dem Testprobenextrakt, um eine Testmischung zu bilden, worin das Immunsuppressivum gewählt ist aus der Gruppe bestehend aus Sirolimus, Tacrolimus, Everolimus, Zotarolimus und Cyclosporin;
(c) Inkubieren der Testmischung unter Bedingungen, die geeignet sind zur Bildung von Komplexen zwischen dem Antikörper und dem Immunsuppressivum, sofern anwesend, welches in der Probe vorhanden ist und immunologisch reaktiv ist mit dem Antikörper; und
(d) Detektieren des Vorhandenseins von jeglichen gebildeten Komplexen.

2. Ein Verfahren zur Einschätzung der Konzentration eines Immunsuppressivums in einer menschlichen Blutprobe umfassend das In-Kontakt-bringen einer menschlichen Blutprobe mit einer extraktiven Reagenz-Zusammensetzung umfassend mindestens 50 Volumenprozent Dimethylsulfoxid, ein Glykol mit von zwei bis sechs Kohlenstoffatomen, und von 30 mM bis 75 mM eines Metallsalzes von Zink, und Wasser, um eine feste Phase und eine Überstandsphase zu erzeugen, Abtrennen der Überstandsphase, und Analysieren der Überstandsphase durch Immunoassay, um die Konzentration eines Immunsuppressivums zu bestimmen, worin das Immunsuppressivum gewählt ist aus der Gruppe bestehend aus Sirolimus, Tacrolimus, Everolimus, Zotarolimus und Cyclosporin.

3. Das Verfahren gemäß Anspruch 1, weiter umfassend das Abtrennen jeglicher festen Phase resultierend aus dem Kontakt der menschlichen Blutprobe mit der extraktiven Reagenz-Zusammensetzung von jeglicher resultierender Überstandsphase, und Analysieren der Überstandsphase hinsichtlich eines Immunsuppressivums.

4. Das Verfahren gemäß irgendeinem der Ansprüche 1 bis 3, worin das Glykol Ethylenglykol (EG), Propylenglykol (PG) oder Mischungen aus EG und PG umfasst.

5. Das Verfahren gemäß irgendeinem der Ansprüche 1 bis 4, worin das Metallsalz von Zink Zinksulfat, Zinkacetat, Zinknitrat, Zinkchlorid oder Mischungen aus zwei oder mehreren dieser Metallsalze umfasst.

6. Das Verfahren gemäß irgendeinem der Ansprüche 1 bis 5, worin die extraktive Reagenz-Zusammensetzung mit der Blutprobe bei einer Temperatur von mindestens 30 Grad Celsius in Kontakt gebracht wird.

7. Das Verfahren gemäß irgendeinem der Ansprüche 1 bis 6, worin die extraktive Reagenz-Zusammensetzung einen Dampfdruck von weniger als Wasserdampfdruck bei 20 Grad Celsius und normalem atmosphärischen Druck hat.

8. Eine extraktive Reagenz-Zusammensetzung für die Extraktion eines Immunsuppressivums aus einer Blutprobe, wobei die Zusammensetzung mindestens 50 Volumenprozent Dimethylsulfoxid, ein Glykol mit von zwei bis sechs Kohlenstoffatomen, und von 30 mM bis 75 mM eines Metallsalzes von Zink, und Wasser umfasst, worin das Immunsuppressivum gewählt ist aus der Gruppe bestehend aus Sirolimus, Tacrolimus, Everolimus, Zotarolimus und Cyclosporin.

9. Die extraktive Reagenz-Zusammensetzung gemäß Anspruch 8, worin das Glykol Ethylenglykol (EG), Propylenglykol (PG) oder Mischungen aus EG und PG umfasst.

10. Die extraktive Reagenz-Zusammensetzung gemäß Ansprüchen 8 oder 9, worin die extraktive Reagenz-Zusammensetzung ein Metallsalz von Zink gewählt aus der Gruppe bestehend aus Zinksulfat, Zinkacetat, Zinknitrat, Zinkchlorid oder Mischungen aus zwei oder mehreren dieser Metallsalze umfasst.

11. Ein Testkit umfassend separate Behälter, die jeweils eine Komponente enthalten, gewählt aus
(a) mindestens einem Antikörper oder Protein, der/das in der Lage ist spezifisch an mindestens ein Immunsuppressivum zu binden, gewählt aus der Gruppe bestehend aus Sirolimus, Tacrolimus, Everolimus, Zotarolimus und Cyclosporin; und
(b) eine extraktive Reagenz-Zusammensetzung umfassend mindestens 50% Dimethylsulfoxid bezogen auf das Volumen der extraktiven Reagenz-Zusammensetzung, ein Glykol mit von zwei bis sechs Kohlenstoffatomen, und von 30 mM bis 75 mM eines Metallsalzes von Zink, und Wasser.

12. Der Testkit gemäß Anspruch 11, worin das Glykol Ethylenglykol (EG), Propylenglykol (PG) oder Mischungen aus EG und PG umfasst.

13. Der Testkit gemäß Ansprüchen 11 oder 12, weiter umfassend einen Behälter, der eine Kontrollzusammensetzung enthält, umfassend mindestens ein Immunsuppressivum gewählt aus der Gruppe bestehend aus Sirolimus, Tacrolimus, Everolimus, Zotarolimus und Cyclosporin.

14. Der Testkit gemäß Ansprüchen 11 - 13, worin das Zinksalz Zinksulfat, Zinkacetat, Zinknitrat oder Zinkchlorid umfasst.

15. Der Testkit gemäß Anspruch 11, worin der Testkit separate Behälter umfasst, die jeweils eine Komponente enthalten, gewählt aus (a) mindestens einem Antikörper oder Protein, der/das in der Lage ist spezifisch an mindestens ein Immunsuppressivum zu binden, gewählt aus der Gruppe bestehend aus Sirolimus, Tacrolimus, Everolimus und Cyclosporin; und (b) einer extraktiven Reagenz-Zusammensetzung weiter umfassend 30% - 33% Ethylenglykol, Propylenglykol oder Mischungen daraus, bezogen auf das Volumen der extraktiven Reagenzzusammensetzung, und umfassend Zinksulfat; und eine Kontrollzusammensetzung umfassend mindestens ein Immunsuppressivum gewählt aus der Gruppe bestehend aus Sirolimus, Tacrolimus, Everolimus, Zotarolimus und Cyclosporin.

## Revendications

1. Méthode d'évaluation de la concentration d'un médicament de la classe des immunosuppresseurs dans un échantillon de sang humain comprenant les étapes de :
(a) combinaison d'une composition de réactifs d'extraction comprenant au moins 50 % en volume de DMSO, un glycol ayant de deux à six atomes de carbone, et de 30 à 75 mM d'un sel métallique de zinc avec un échantillon de sang humain et de l'eau pour former un extrait d'échantillon d'essai ;
(b) combinaison d'au moins un anticorps ou d'une protéine capable de se lier à un médicament de la classe des immunosuppresseurs avec l'extrait d'échantillon d'essai pour former un mélange d'essai, dans laquelle le médicament de la classe des immunosuppresseurs est choisi dans le groupe constitué par le sirolimus, le tacrolimus, l'évérolimus, le zotarolimus et la cyclosporine ;
(c) incubation du mélange d'essai dans des conditions convenables pour la formation de complexes entre l'anticorps et le médicament de la classe des immunosuppresseurs, le cas échéant, qui est présent dans l'échantillon et réagit immunologiquement avec l'anticorps ; et
(d) détection de la présence de tout complexe susceptible de s'être formé.

2. Méthode d'évaluation de la concentration d'un médicament de la classe des immunosuppresseurs dans un échantillon de sang humain comprenant la mise en contact d'un échantillon de sang humain avec une composition de réactifs d'extraction comprenant au moins 50 % en volume de diméthylsulfoxyde, un glycol ayant de deux à six atomes de carbone, et de 30 à 75 mM d'un sel métallique de zinc et de l'eau pour former une phase solide et une phase surnageant, la séparation de la phase surnageant, et l'analyse de la phase surnageant par dosage immunologique pour déterminer la concentration d'un médicament de la classe des immunosuppresseurs, dans laquelle le médicament de la classe des immunosuppresseurs est choisi dans le groupe constitué par le sirolimus, le tacrolimus, l'évérolimus, le zotarolimus et la cyclosporine.

3. Méthode selon la revendication 1 comprenant en outre la séparation de toute phase solide résultant du contact de l'échantillon de sang humain avec la composition de réactifs d'extraction de toute phase surnageant obtenue et l'analyse de la phase surnageant pour identifier un médicament de la classe des immunosuppresseurs.

4. Méthode selon l'une quelconque des revendications 1 à 3 dans laquelle le glycol comprend l'éthylène glycol (EG), le propylène glycol (PG) ou des mélanges d'EG et PG.

5. Méthode selon l'une quelconque des revendications 1 à 4 dans laquelle le sel métallique de zinc comprend le sulfate de zinc, l'acétate de zinc, le nitrate de zinc, le chlorure de zinc, ou des mélanges de deux de ces sels métalliques ou plus.

6. Méthode selon l'une quelconque des revendications 1 à 5 dans laquelle la composition de réactifs d'extraction est mise en contact avec l'échantillon de sang à une température d'au moins 30°C.

7. Méthode selon l'une quelconque des revendications 1 à 6 dans laquelle la composition de réactifs d'extraction a une pression de vapeur inférieure à la pression de vapeur d'eau à 20°C et à pression atmosphérique normale.

8. Composition de réactifs d'extraction pour extraire un médicament de la classe des immunosuppresseurs d'un échantillon de sang, la composition comprenant au moins 50 % en volume de diméthylsulfoxyde, un glycol ayant de deux à six atomes de carbone, et de 30 à 75 mM d'un sel métallique de zinc et de l'eau, dans laquelle le médicament de la classe des immunosuppresseurs est choisi dans le groupe constitué par le sirolimus, le tacrolimus, l'évérolimus, le zotarolimus et la cyclosporine.

9. Composition de réactifs d'extraction selon la revendication 8 dans laquelle le glycol comprend l'éthylène glycol (EG), le propylène glycol (PG) ou des mélanges d'EG et PG.

10. Composition de réactifs d'extraction selon les revendications 8 ou 9 dans laquelle la composition de réactifs d'extraction comprend un sel métallique de zinc choisi dans le groupe constitué par le sulfate de zinc, l'acétate de zinc, le nitrate de zinc, le chlorure de zinc, ou des mélanges de deux de ces sels métalliques ou plus.

11. Kit d'essai comprenant des récipients distincts contenant chacun un composant choisi parmi
(a) au moins un anticorps ou une protéine capable de se lier spécifiquement à au moins un médicament de la classe des immunosuppresseurs choisi dans le groupe constitué par le sirolimus, le tacrolimus, l'évérolimus, le zotarolimus et la cyclosporine ; et
(b) une composition de réactifs d'extraction comprenant au moins 50 % de diméthylsulfoxyde en volume de la composition de réactifs d'extraction, un glycol ayant de deux à six atomes de carbone, et de 30 à 75 mM d'un sel métallique de zinc et de l'eau.

12. Kit d'essai selon la revendication 11 dans lequel le glycol comprend l'éthylène glycol (EG), le propylène glycol (PG) ou des mélanges d'EG et PG.

13. Kit d'essai selon les revendications 11 ou 12 comprenant en outre un récipient contenant une composition témoin comprenant au moins un médicament de la classe des immunosuppresseurs choisi dans le groupe constitué par le sirolimus, le tacrolimus, l'évérolimus, le zotarolimus et la cyclosporine.

14. Kit d'essai selon les revendications 11 à 13 dans lequel le sel de zinc comprend le sulfate de zinc, l'acétate de zinc, le nitrate de zinc ou le chlorure de zinc.

15. Kit d'essai selon la revendication 11, dans lequel ledit kit d'essai comprend des récipients distincts contenant chacun un composant choisi parmi (a) au moins un anticorps ou une protéine capable de se lier spécifiquement à au moins un médicament de la classe des immunosuppresseurs choisi dans le groupe constitué par le sirolimus, le tacrolimus, l'évérolimus, le zotarolimus et la cyclosporine ; et (b) une composition de réactifs d'extraction comprenant en outre de 30 à 33 % d'éthylène glycol, de propylène glycol ou des mélanges de ceux-ci en volume de la composition de réactifs d'extraction, et comprenant du sulfate de zinc ; et une composition témoin comprenant au moins un médicament de la classe des immunosuppresseurs choisi dans le groupe constitué par le sirolimus, le tacrolimus, l'évérolimus, le zotarolimus et la cyclosporine.
